# EUROPEAN PATENT SPECIFICATION

(11) **EP 1 087 005 B1**
(45) Date of publication and mention of the grant of the patent: **25.02.2004**
(21) Application number: 00810847.4
(22) Date of filing: 19.09.2000
(51) Int. Cl.: C09K 11/06, C08K 5/3415, C07D 487/04, C09B 57/00

(54) **Fluorescent diketopyrrolopyrroles**
Fluoreszente Diketopyrrolopyrrolen
Dicétopyrrolopyrroles fluorescentes

(30) Priority: 27.09.1999 EP 99810867
(43) Date of publication of application: 28.03.2001
(73) Proprietor: Ciba Specialty Chemicals Holding Inc., 4057 Basel (CH)
(72) Inventor: Moretti, Robert, 1213 Petit-Lancy (CH); Hao, Zhimin, 4125 Riehen (CH); Yamamoto, Hiroshi, Takarazuka-shi, Hyogo 665-0864 (JP)

(56) References cited:
- EP-A- 0 133 156
- EP-A- 0 353 184
- EP-A- 0 811 625
- WO-A-96/08537
- WO-A-98/25927
- WO-A-98/32802
- WO-A-98/33864
- PATENT ABSTRACTS OF JAPAN vol. 015, no. 122 (P-1184), 26 March 1991 (1991-03-26) & JP 03 011357 A (DAINIPPON INK & CHEM INC), 18 January 1991 (1991-01-18)

## Description

The present invention relates to fluorescent diketopyrrolopyrroles ("DPPs") of the formula I 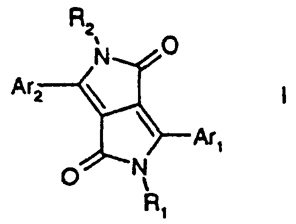 wherein R₁ and R₂, independently from each other, stand for C₁-C₂₅-alkyl, allyl which can be substituted one to three times with C₁-C₃alkyl or Ar₃, -CR₃R₄-(CH₂)ₘ-Ar₃, wherein R₃ and R₄ independently from each other stand for hydrogen or C₁-C₄alkyl, or phenyl which can be substituted one to three times with C₁-C₃ alkyl,
Ar₃ stands for phenyl or 1- or 2-naphthyl which can be substituted one to three times with C₁-C₈alkyl, C₁-C₈alkoxy, halogen or phenyl, which can be substituted with C₁-C₈alkyl or C₁-C₈alkoxy one to three times, and m stands for 0, 1, 2, 3 or 4, and wherein C₁-C₂₅-alkyl or -CR₃R₄-(CH₂)ₘ-Ar₃, preferably C₁-C₂₅-alkyl, can be substituted with a functional group capable of increasing the solubility in water which is selected from a tertiary amino group, -SO₃⁻, or PO₄²⁻, Ar₁ and Ar₂, independently from each other, stand for 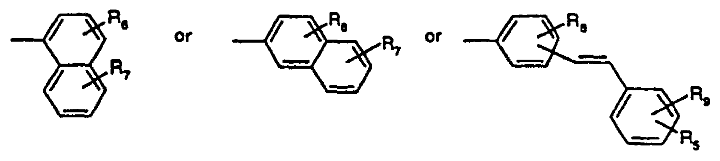 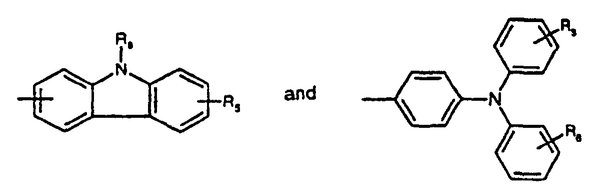 wherein
R₅ stands for C₁-C₈alkyl, -NR₈R₉, -OR₁₀, -S(O)ₙR₈, -Se(O)ₙR₈, or phenyl, which can be substituted one to three times with C₁-C₈alkyl or C₁-C₈alkoxy,
wherein R₈ and R₉, independently from each other, stand for hydrogen, C₁-C₂₅-alkyl, C₅-C₁₂-cycloalkyl, -CR₃R₄-(CH₂)ₘ-Ph, R₁₀, wherein R₁₀ stands for C₆-C₂₄-aryl, or a saturated or unsaturated heterocyclic radical comprising five to seven ring atoms, wherein the ring consists of carbon atoms and one to three hetero atoms selected from the group consisting of nitrogen, oxygen and sulfur, wherein Ph, the aryl and heterocyclic radical can be substituted one to three times with C₁-C₈alkyl, C₁-C₈alkoxy, or halogen, or R₈ and R₉ stand for -C(O)R₁₁, wherein R₁₁ can be C₁-C₂₅-alkyl, C₅-C₁₂-cycloalkyl, R₁₀, -OR₁₂ or -NR₁₃R₁₄, wherein R₁₂, R₁₃, and R₁₄ stand for C₁-C₂₅-alkyl, C₅-C₁₂-cycloalkyl, C₆-C₂₄-aryl,
or
a saturated or unsaturated heterocyclic radical comprising five to seven ring atoms, wherein the ring consists of carbon atoms and one to three hetero atoms selected from the group consisting of nitrogen, oxygen and sulfur, wherein the aryl and heterocyclic radical can be substituted one to three times with C₁-C₈alkyl or C₁-C₈alkoxy, or -NR₈R₉ stands for a five- or sixmembered heterocyclic radical in which R₈ and R₉ together stand for tetramethylene, pentamethylene, -CH₂-CH₂-O-CH₂-CH₂-, or -CH₂-CH₂-NR₅-CH₂-CH₂-, preferably -CH₂-CH₂-O-CH₂-CH₂-, and n stands for 0, 1, 2 or 3,
and wherein R₆ and R₇, independently from each other, stand for hydrogen or R₅, but do not stand simultaneously for hydrogen, preferably R₆ stands for R₅ and R₇ for hydrogen.

US 4,579,949 describes a process for the preparation of DPPs which are unsubstituted at the nitrogen atoms of the pyrrolo-rings. Especially example 45 describes the DPP-compound of the formula II 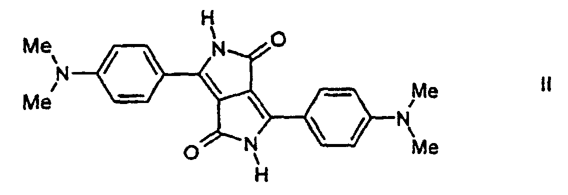 DPP-compound II, however, is violet, exhibits only insufficient fluorescence and solubility.

Further, EP-A 133,156 claims generically DPP-compounds, however, compounds of formula I are not mentioned explicitly and no teaching is given that DPP-compounds of formula I could exhibit a red or orange fluorescence.

EP-A 499,011 describes electroluminescent devices comprising DPP-compounds. Particularly, in example 1 the DPP-derivative of formula III 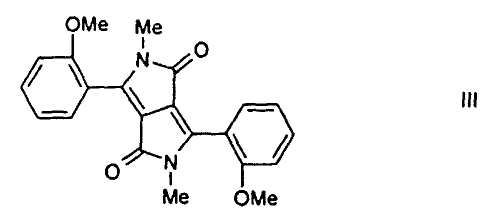 is disclosed. However, no teaching is given with regard to the fluorescence of DPP-compounds and a way to obtain DPP-compounds exhibiting a red or orange fluorescence.

WO 98/33862 describes the use of the DPP-compound of formula IV 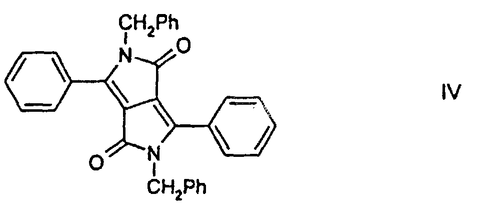 as a guest molecule in electroluminescent devices. However, no teaching is given with regard to the fluorescence of DPP-compounds and a way to obtain DPP-compounds exhibiting a red or orange fluorescence.

WO98/25927 relates to liquid crystalline N-substituted 1,4-diketo-3,6-diaryl-pyrrolo[3,4-c]pyrroles, 1,4-diketo-3,6-dialkylpyrrolo[3,4-c]pyrroles and 1,4-diketo-3-alkyl-6-aryl-pyrrolo[3,4-c]pyrroles.

EP-A-0 811 625 relates to a process for preparing diketopyrrolopyrrole derivatives. In Example 8 of D2 the preparation of 2,5-di(hydroxymethyl)-3,6-di(biphen-1-yl)pyrrolo[3,4-c]pyrrole-1,4-dione is disclosed. The diketopyrrolopyrrole derivatives prepared according to the process of D2 are used as rheology-improving agents (see D2, page 13, lines 24 to 27).

WO96/08537 relates to a process for producing N-methylated organic pigments. In Example 3 of D3 the preparation of N,N'-dimethyl-p-diphenyl-1,4-diketo-pyrrolo[3,4-c]-pyrrole is disclosed.

WO98/33864 relates to a solid composition comprising at least one host chromophore selected from the group consisting of a benzo[4,5] imidazo[2,1-a]isoindol-11-ones and an effective amount of at least one guest chromophore, and if desired a polymer C, wherein the emission spectrum of the host chromophore overlaps with the absorption spectrum of the guest chromophore, and wherein
(a1) the host chromophore is covalently linked to a polymer backbone A ("host polymer"), and/or
(a2) the guest chromophore is covalently linked to a polymer backbone B ("guest polymer"). According to page 41, last paragraph of D4 examples of guest molecules used for composition (a1) are di-(p-pheny)phenyl N,N'-dibutyl diketo pyrrolo [3,4-c] pyrrole and di-(p-methyl)phenyl N,N'-dibutyl diketo pyrrolo [3,4-c] pyrrole.

JP-A-03011357 relates to electrophotographic photosensitive body comprising a disazo compound of the formula 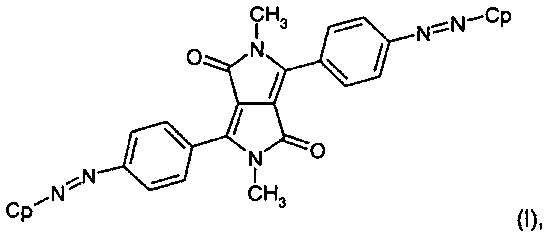 wherein Cp denotes a residual coupler group. By incorporating the disazo compound of D4 into the photosensitive body an electrophotographic body with excellent durability and high sensitivity is obtained.

In addition, commercially available red fluorescent dyes such as thioindigo derivatives do not show superior light stability when incorporated in plastics. In addition, commercially available red fluorescent dyes cannot be applied to colour polyamides, because they decompose during the manufacturing process.

Hence, the object of this invention was to provide red or orange fluorescent compounds with a high heat stability, a good solubility in polymers, hydrocarbon based fuels, lubricants, and water, a high light stability, and the ability to be used in plastics, especially polyamides, without decomposition and loss of lightfastness, and in paints.

Accordingly, the abovementioned DPP-compounds I were found.

In addition, a process for its preparation and its use were found, too.

Red or orange fluorescent compounds means that the inventive compounds preferably have a fluorescence emission maximum in the range of from 520 to 780, more preferably from 550 to 700, more preferred from 580 to 650 nm. Further, the inventive compounds preferably exhibit an absorption in the range of 480 to 580 nm.

The inventive compounds I usually exhibit a fluorescence quantum yield ("FQY") in the range of from 1 > FQY ≥ 0.3 (measured in aerated toluene or DMF). Further, in general, the inventive compounds I exhibit a molar absorption coefficient in the range of from 5000 to 100000.

A preferred embodiment relates to DPP-compounds I, wherein R₁ = R₂, and R₅ = R₆, R₇ = hydrogen, and Ar₁ = Ar₂, particularly preferred wherein in addition to the above R₃ = R₄ = H, m = 0 and n = 0, most preferred are DPP-compounds in which
(a) R₁ = R₂ = C₁-C₈alkyl, Ar₁ = Ar₂ = stilbene, and R₈ = R₉ = C₁-C₈alkyl or phenyl, or
(b) R₁ = R₂ = C₁-C₈alkyl, -(CH₂)ₘ-Ph, Ar₁ = Ar₂ = stilbene, and R₈ = C₁-C₈alkyl, phenyl or a heterocyclic radical, both unsubstituted or substituted, or C₅-C₁₂-cycloalkyl, or
(c) R₁ = R₂ = -CH₂-Ph, wherein phenyl can be substituted with phenyl, naphthyl or C₁-C₄alkyl up to two times, Ar₁ = Ar₂ = 1- or 2-naphthyl, R₅ = R₆ = R₇ = hydrogen, in case where Ar₁ = Ar₂ = 1- or 2-naphthyl, or, in all other cases, C₁-C₈alkyl or phenyl.

Particularly preferred DPP-compounds I are the following compounds: 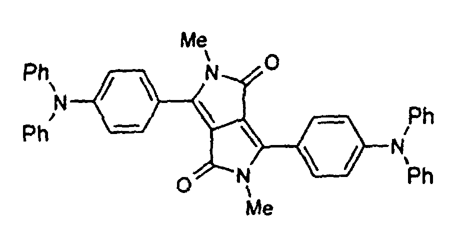 C₁-C₂₅alkyl is typically linear or branched - where possible - methyl, ethyl, n-propyl, isopropyl, n-butyl, sec.-butyl, isobutyl, tert.-butyl, n-pentyl, 2-pentyl, 3-pentyl, 2,2-dimethylpropyl, n-hexyl, n-heptyl, n-octyl, 1,1,3,3-tetramethylbutyl and 2-ethylhexyl, n-nonyl, decyl, undecyl, dodecyl, tetradecyl, pentadecyl, hexadecyl, heptadecyl, octadecyl, eicosyl, heneicosyl, docosyl, tetracosyl or pentacosyl, preferably C₁-C₈alkyl such as methyl, ethyl, n-propyl, isopropyl, n-butyl, sec.-butyl, isobutyl, tert.-butyl, n-pentyl, 2-pentyl, 3-pentyl, 2,2-dimethylpropyl, n-hexyl, n-heptyl, n-octyl, 1,1,3,3-tetramethylbutyl and 2-ethylhexyl, more preferably C₁-C₄alkyl such as typically methyl, ethyl, n-propyl, isopropyl, n-butyl, sec.-butyl, isobutyl, tert.-butyl; C₁-C₃alkyl stands for methyl, ethyl, n-propyl, or isopropyl; C₁-C₆alkyl stands for methyl, ethyl, n-propyl, isopropyl, n-butyl, sec-butyl, isobutyl, tert.-butyl, n-pentyl, 2-pentyl, 3-pentyl, 2,2-dimethyl-propyl, or n-hexyl.

C₁-C₈alkoxy is typically methoxy, ethoxy, n-propoxy, isopropoxy, n-butoxy, sec.-butoxy, isobutoxy, tert.-butoxy, n-pentoxy, 2-pentoxy, 3-pentoxy, 2,2-dimethylpropoxy, n-hexoxy, n-heptoxy, n-octoxy, 1,1,3,3-tetramethylbutoxy and 2-ethylhexoxy, preferably C₁-C₄alkoxy such as typically methoxy, ethoxy, n-propoxy, isopropoxy, n-butoxy, sec.-butoxy, isobutoxy, tert.-butoxy.

C₆-C₂₄aryl is typically phenyl, 1-naphthyl, 2-naphthyl, 4-biphenyl, phenanthryl, 2- or 9-fluorenyl or anthracenyl, preferablyC₆-C₁₂aryl such as phenyl, 1-naphthyl, 2-naphthyl, 4-biphenyl.

C₇-C₂₄aralkyl is typically benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl, ω-phenyl-octadecyl, ω-phenyl-eicosyl or ω-phenyl-docosyl, preferably C₇-C₁₈aralkyl such as benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl or ω-phenyl-octadecyl, and particularly preferred C₇-C₁₂aralkyl such as benzyl, 2-benzyl-2-propyl, β-phenyl-ethyl, α,α-dimethylbenzyl, ω-phenyl-butyl, or ω,ω-dimethyl-ω-phenyl-butyl.

C₅-C₁₂cycloalkyl is typically cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, cyclooctyl.

Heteroaryl with five to seven ring atoms, wherein nitrogen, oxygen or sulfur are the possible hetero atoms, is typically an unsaturated heterocyclic radical with five to 18 atoms having at least six conjugated π-electrons such as thienyl, benzo[b]thienyl, dibenzo[b,d]thienyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, perimidinyl, phenanthrolinyl, phenazinyl, isothiazolyl, phenothiazinyl, isoxazolyl, furazanyl or phenoxazinyl, preferably the abovementioned mono- or bicyclic heterocyclic radicals.

The inventive DPP-compounds I can be synthesized according to methods well known in the art such as described in EP-A 133,156, e.g. in analogy to example 15.

A preferred embodiment of this invention relates to a process for the preparation of the inventive compounds I by treating in a first step the DPP derivative of formula V, 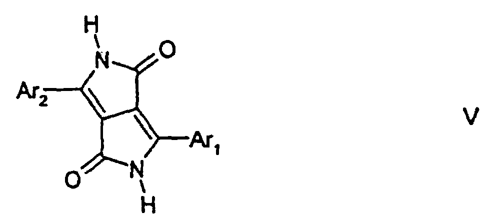 , wherein Ar₁ and Ar₂ are defined as above, with a base, then, in a second step, treating the reaction mixture obtained in the first step with a alkylating agent, wherein in the first step the base is a hydride, an alkali metal alkoxide or a carbonate, and the alkylating agent is a sulfonate, tosylate, mesylate, carbonate, sulfate, or halogen compound of the formula (R₁)_{1 or 2}X, wherein X stands for SO₃-, (p-Me-phenyl)SO₂-, (2,4,6-trimethyl-phenyl)SO₂-, -CO₃-, -SO₄-, or halogen such as chlorine, bromine or iodine, preferably chlorine, bromine or iodine, particularly preferred for bromine or iodine, or a mixture of (R₁)_{1 or 2}X and (R₂)_{1 or 2}X wherein R¹ and R² are defined as above.

As a hydride usually an alkali metal hydride such as sodium hydride, lithium hydride, or potassium hydride, as an alkali metal alkoxide in general an alkali metal C₁-C₄alkoxide such as sodium or potassium tert. butoxide, sodium tert.-amylate, and as a carbonate usually sodium or potassium carbonate can be used, preferably sodium hydride.

Usually, the first step of the preferred preparation of compound I starting from compound V is carried out at a temperature in the range of from -25 to 100, preferably from 0 to 25°C. Preferably, the reaction is carried out in the presence of a solvent, preferably a dipolar aprotic solvent such as carboxamides, lactams, urea derivatives, sulfones and nitrobenzene such as dimethyl formamide ("DMF"), dimethyl acetamide ("DMA"), N-methylpyrrolidone ("NMP"), N,N'-dimethylethylene urea and N,N'-dimethylpropylene urea.

In case a solvent is used, a weight ratio of solvent to DPP-compound is chosen in the range of from 100:1 to 5:1, preferably from 25:1 to 10:1.
In addition, it is preferred to carry out the first step in the presence of a phase transfer catalyst such as a tetra alkyl ammonium halide such as tetraethyl ammonium bromide.

Usually, a molar ratio of base to DPP-compound V is chosen in the range of from 10:1 to 2:1, preferably from 4:1 to 2:1.

Preferably, a molar ratio of DPP-compound V to the phase transfer catalyst is chosen in the range of from 100:1 to 5:1, preferably from 25:1 to 10:1.

Generally, the reaction time depends *inter alia* on the reactivity of the chosen reactants and the chosen temperature. As an example, if room temperature is chosen as reaction temperature, a reaction time is as a rule in the range of from 0.5 to 24 hours.

Preferably, the halogen compound R₁-X (or the aforementioned mixture) is added to the reaction mixture obtained in the first step in the same solvent used in the first step.

The reaction temperature in the second process step usually is chosen in the range of from 0 to 160, preferably from 25 to 110°C, depending on *inter alia* the desired reaction pressure and solvent used. The reaction time generally is chosen in the range of from 0.5 to 120, preferably from 12 to 60 hours.

As a rule the molar ratio of R₁-X to DPP compound V is chosen in the range of from 10:1 1 to 2:1, preferably from 4:1 to 2:1.

In case a solvent is used, the amount of solvent usually is chosen in the range of from 100:1 to 5:1, preferably from 25:1 to 10:1, based on the amount of halogen compound R₁-X.
Further, preferably the same solvent is used as in the first step, if a solvent is used in the first step. If no solvent is used in the first step, the same solvents can be used as mentioned above.

The obtained reaction mixture can be worked up by applying methods well known in the art, e.g. by precipitating the product in the presence of an appropriate solvent such as water, and, if deemed necessary, by re-crystallization in an appropriate solvent such as ethanol.

Other methods for example are the addition of an alcohol to quench the excess base followed by filtration.

Compounds V are described e.g. in US 4,579,949, and/or can be prepared according to the method described therein, in which an appropriate nitrile is reacted with a corresponding dialkyl or diaryl succinate, e.g. NC-Ar₁ is reacted with sodium tert.-amyl alcohol followed by the addition of diisopropyl succinate. This method is preferred in case Ar₁ and/or Ar₂ stand for a biphenyl radical (i.e. R₅ and/or R₆ stand for phenyl or substituted phenyl in 4-position), or for the compounds described below (DPP VI).

Compounds I are also available in analogy to the method described in EP-A 353,184, which comprises reacting a DPP-compound of formula VI 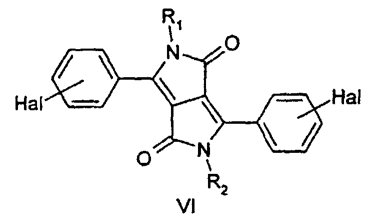 wherein R₁ and R₂ are defined as above, Hal stands for halogen such as fluorine, chlorine, bromine or iodine, preferably chlorine or bromine, with a nucleophilic agent such as a secondary amine, HNR₈R₉, a thiol, HSR₈, or HS(O)ₙR₈, an alcohol, HOR₁₀, a diselenide, R₈(O)ₙSe-Se(O)ₙR₈, preferably in a molar ratio of DPP VI:nucleophilic agent in the range of 1.2:1 to 0.8:1, or, if R₂ has the same meaning as R₁ in the range of from 1:2.5 to 1:1, in the presence of an anhydrous dipolar aprotic solvent, and of an anhydrous base in an amount in the range of from usually 0.1 to 15 moles per mole of the nucleophilic agent, at a temperature in the range of from usually 100 to 220°C and under a pressure generally in the range of from 100 to 300 kPa.

Examples of suitable anhydrous dipolar aprotic solvents are carboxamides, lactams, urea derivatives, sulfones and nitrobenzene such as DMF, DMA, NMP, N,N'-dimethylethylene urea and N,N'-dimethylpropylene urea.

Suitable anhydrous bases are e.g. anhydrous organic bases such as quinoline, or preferably, an excess of the secondary amine used for the amination, the aforementioned carbonates such as sodium or potassium carbonate and alkali metal hydrides such as sodium hydride. In case a diselenide, R₇(O)ₙSe-Se(O)ₙR₇, is used, an alkali metal hydride, preferably sodium hydride, has to be used as a base.

The corresponding 1 - and 2-naphthyl-derivatives can be prepared analogously.

DPP-compounds VI are known and/or can be prepared e.g. according to the method described in US 4,579,949, which methods comprises reacting a dialkyl or diaryl succinate with a nitrile, e.g. dimethyl succinate can be reacted with p-chloro benzonitrile according to example 6 in US 4,579,949 to yield the corresponding DPP compound VI, in which Hal stands for chlorine.

Compounds R₁-X are commercially available or can be prepared by methods well known in the art.

A further embodiment of the invention on hand concerns a process for the preparation of the inventive compounds I
(a) in treating in a first step the DPP derivative of formula VI, wherein R₁ and R₂ are hydrogen, with a nucleophilic agent such as a secondary amine, HNR₈R₉, a thiol, HSR₈, or HS(O)ₙR₈, an alcohol, HOR₁₀, a diselenide, R₈(O)ₙSe-Se(O)ₙR₈, preferably in a molar ratio of DPP Vl:nucleophilic agent in the range of 1.2:1 to 0.8:1, or, if R₂ has the same meaning as R₁ in the range of from 1:2.5 to 1:1, in the presence of an anhydrous dipolar aprotic solvent, and of an anhydrous base in an amount in the range of from usually 0.1 to 15 moles per mole of the nucleophilic agent, at a temperature in the range of from usually 100 to 220°C and under a pressure generally in the range of from 100 to 300 kPa, and optionally isolating the obtained compound V,
(b) then treating the obtained compound V, with a base, thereafter in a second step, treating the reaction mixture obtained in the first step of (b) with an alkylating agent, wherein in the first step of (b) the base is a hydride, an alkali metal alkoxide or a carbonate, and the alkylating agent is a sulfonate, tosylate, mesylate, carbonate, sulfate, or halogen compound of the formula (R₁)₁ or ₂X, wherein X stands for SO₃-, (p-Me-phenyl)SO₂-, (2,4,6-trimethylphenyl)SO₂-, -CO₃-, -SO₄-, or halogen, or a mixture of (R₁)_{1 or 2}X and (R₂)_{1 or 2}X (it is evident, that the number of R₁-units (either one or two) in (R₁)_{1 or 2}X depends on the nature of the chosen rest X, i.e. there can be only two R₁-units if X stands for a divalent anion such as -CO₃-, -SO₄- etc.).

Water-soluble compounds I, i.e. inventive compounds I being substituted with a functional group capable of increasing the solubility in water such as a tertiary amino group, SO₃⁻, or PO₄²⁻, can be prepared by using well-known methods in the art. The following routes are representative examples, and, hence, do not restrict the invention just to these examples: 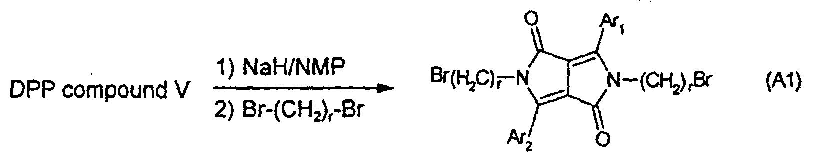 wherein r stands for an integer from usually 2 to 25; instead of linear alkyl groups, one could also use branched alkyl groups or aralkyl groups such as Br-(CH₂)ᵣ₁-aryl-(CH₂)ᵣ₂-Br, r₁ and r₂ usually being whole numbers in the range of from 0 to 10; 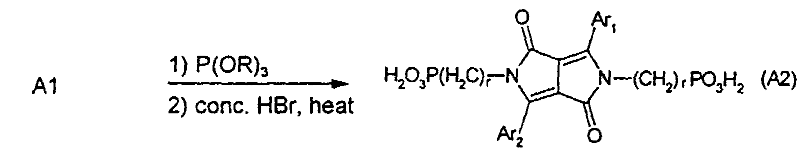 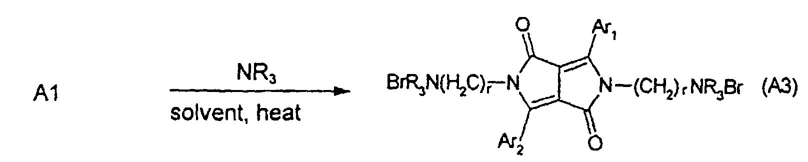 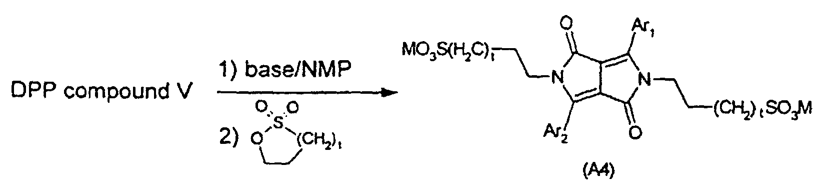 wherein M stands for a metal ion such as sodium or potassium, and t is 1 or 2.

Accordingly, the present invention also relates to fluorescent diketopyrrolopyrroles, 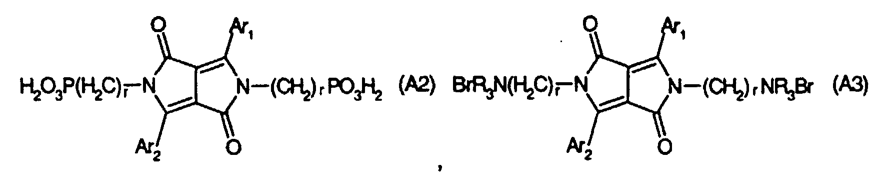 or 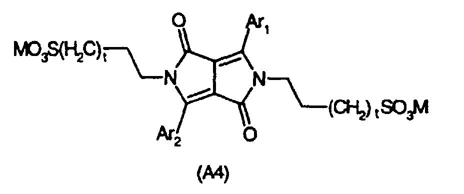 wherein Ar₁ and Ar₂ are defined as above, r stands for an integer from 2 to 25; instead of linear alkyl groups, wherein the group -(CH₂)ᵣ- could be replaced by branched alkyl groups or aralkyl groups, such as -(CH₂)ᵣ₁-aryl-(CH₂)ᵣ₂-, r₁ and r₂ being whole numbers in the range of from 0 to 10; wherein M stands for a metal ion such as sodium or potassium, and t is 1 or 2.

Another embodiment of the present invention is related to a method of coloring high molecular weight organic materials (having a molecular weight usually in the range of from 10³ to 10⁷ g/mol) by incorporating the inventive fluorescent DPP compounds I by known methods in the art.

As high molecular weight organic materials the following can be used such as biopolymers, and plastic materials, including fibres.

The present invention relates preferably to the use of the inventive DPPs I for the preparation of
inks, for printing inks in printing processes, for flexographic printing, screen printing, packaging printing, security ink printing, intaglio printing or offset printing, for pre-press stages and for textile printing, for office, home applications or graphics applications, such as for paper goods, for example, for ballpoint pens, felt tips, fiber tips, card, wood, (wood) stains, metal, inking pads or inks for impact printing processes (with impact-pressure ink ribbons), for the preparation of
colorants, for coating materials, for industrial or commercial use, for textile decoration and industrial marking, for roller coatings or powder coatings or for automotive finishes, for high-solids (low-solvent), water-containing or metallic coating materials or for pigmented formulations for aqueous paints, for the preparation of
pigmented plastics for coatings, fibers, platters or mold carriers, for the preparation of non-impact-printing material for digital printing, for the thermal wax transfer printing process, the ink jet printing process or for the thermal transfer printing process, and also for the preparation of
color filters, especially for visible light in the range from 400 to 700 nm, for liquid-crystal displays (LCDs) or charge combined devices (CCDs) or for the preparation of
cosmetics or for the preparation of polymeric ink particles, toners, dye lasers, dry copy toners liquid copy toners, or electrophotographic toners, and electroluminescent devices.

Illustrative examples of suitable organic materials of high molecular weight which can be colored with the inventive fluorescent DPPs I of this invention are vinyl polymers, for example polystyrene, poly-α-methylstyrene, poly-p-methylstyrene, poly-p-hydroxystyrene, poly-p-hydroxyphenylstyrene, polymethyl methacrylate and polyacrylamide as well as the corresponding methacrylic compounds, polymethylmaleate, polyacrylonitrile. polymethacrylonitrile, polyvinyl chloride, polyvinyl fluoride, polyvinylidene chloride, polyvinylidene fluoride, polyvinyl acetate, polymethyl vinyl ether and polybutyl vinyl ether; polymers which are derived from maleinimide and/or maleic anhydride, such as copolymers of maleic anhydride with styrene; polyvinyl pyrrolidone; ABS; ASA; polyamides; polyimides; polyamidimides; polysulfones; polyether sulfones; polyphenylene oxides; polyurethanes; polyureas; polycarbonates; polyarylenes; polyarylene sulfides; polyepoxides; polyolefins such as polyethylene and polypropylene; polyalkadienes; biopolymers and the derivatives thereof e.g. cellulose, cellulose ethers and esters such as ethylcellulose, nitrocellulose, cellulose acetate and cellulose butyrate, starch, chitin, chitosan, gelatin, zein; natural resins; synthetic resins such as alkyd resins, acrylic resins, phenolic resins, epoxide resins, aminoformaldehyde resins such as urea/formaldehyde resins and melamine/formaldehyde resin; vulcanized rubber; casein; silicone and silicone resins; rubber, chlorinated rubber, and also polymers which are used, for example, as binders in paint systems, such as novolaks which are derived from C₁-C₆-aldehydes such as formaldehyde and acetaldehyde and a binuclear or mononuclear, preferably mononuclear, phenol which, if desired, is substituted by one or two C₁-C₉alkyl groups, one or two halogen atoms or one phenyl ring, such as o-, m- or p-cresol, xylene, p-tert.-butylphenol, o-, m- or p-nonylphenol, p-chlorophenol or p-phenylphenol, or a compound having more than one phenolic group such as resorcinol, bis(4-hydroxyphenyl)methane or 2,2-bis(4-hydroxyphenyl)propane; as well as suitable mixtures of said materials.

Particularly preferred high molecular weight organic materials, in particular for the preparation of a paint system, a printing ink or ink, are, for example, cellulose ethers and esters, e.g. ethylcellulose, nitrocellulose, cellulose acetate and cellulose butyrate, natural resins or synthetic resins (polymerization or condensation resins) such as aminoplasts, in particular urea/formaldehyde and melamine/formaldehyde resins, alkyd resins, phenolic plastics, polycarbonates, polyolefins, polystyrene, polyvinyl chloride, polyamides, polyurethanes, polyester, ABS, ASA, polyphenylene oxides, vulcanized rubber, casein, silicone and silicone resins as well as their possible mixtures with one another.

It is also possible to use high molecular weight organic materials in dissolved form as film formers, for example boiled linseed oil, nitrocellulose, alkyd resins, phenolic resins, melamine/formaldehyde and urea/formaldehyde resins as well as acrylic resins.

Said high molecular weight organic materials may be obtained singly or in admixture, for example in the form of granules, plastic materials, melts or in the form of solutions, in particular for the preparation of spinning solutions, paint systems, coating materials, inks or printing inks.

In a particularly preferred embodiment of this invention, the inventive fluorescent DPPs I are used for the mass coloration of polyvinyl chloride, polyamides and, especially, polyolefins such as polyethylene and polypropylene as well as for the preparation of paint systems, including powder coatings, inks, printing inks, color filters and coating colors.
Illustrative examples of preferred binders for paint systems are alkyd/melamine resin paints, acryl/melamine resin paints, cellulose acetate/cellulose butyrate paints and two-pack system lacquers based on acrylic resins which are crosslinkable with polyisocyanate.

According to observations made to date, the inventive fluorescent DPPs I can be added in any desired amount to the material to be colored, depending on the end use requirements. In the case of high molecular weight organic materials, for example, the fluorescent DPPs I prepared according to this invention can be used in an amount in the range from 0.01 to 40, preferably from 0.01 to 5% by weight, based on the total weight of the colored high molecular weight organic material.

Hence, another embodiment of the present invention relates to a composition comprising
(a) 0.01 to 50, preferably 0.01 to 5, particularly preferred 0.01 to 2% by weight, based on the total weight of the colored high molecular organic material, of a fluorescent DPP I according to the present invention, and
(b) 99.99 to 50, preferably 99.99 to 95, particularly preferred 99.99 to 98% by weight, based on the total weight of the colored high molecular organic material, of a high molecular organic material, and
(c) if desired, customary additives such as rheology improvers, dispersants, fillers, paint auxiliaries, siccatives, plasticizers, UV-stabilizers, and/or additional pigments or corresponding precursors in effective amounts, such as e.g. from 0 to 50% by weight, based on the total weight of (a) and (b).

To produce non-brittle mouldings or to diminish their brittleness, so-called plasticizers can be added to the high molecular weight organic materials prior to moulding. Plasticizers may be, for example, esters of phosphoric acid, phthalic acid and sebacic acid. Said plasticizers may be added before, during or after pigmenting the high molecular weight organic materials with the inventive fluorescent DPPs I.

To obtain different shades, the inventive fluorescent DPPs I may advantageously be used in admixture with fillers, transparent and opaque white, colored and/or black pigments as well as customary luster pigments in the desired amount.

For the preparation of paints systems, coating materials, color filters, inks and printing inks, the corresponding high molecular weight organic materials, such as binders, synthetic resin dispersions etc. and the inventive fluorescent DPPs I are usually dispersed or dissolved together, if desired together with customary additives such as dispersants, fillers, paint auxiliaries, siccatives, plasticizers and/or additional pigments or pigment precursors, in a common solvent or mixture of solvents. This can be achieved by dispersing or dissolving the individual components by themselves, or also several components together, and only then bringing all components together, or by adding everything together at once.

Hence, a further embodiment of the present invention relates to a method of using the inventive fluorescent DPPs I for the preparation of dispersions and the corresponding dispersions, and paint systems, coating materials, color filters, inks and printing inks comprising the inventive fluorescent DPPs I.

A particularly preferred embodiment relates to the use of the inventive DPPs I for the preparation of fluorescent tracers for e.g. leak detection of fluids such as lubricants, cooling systems etc., as well as to fluorescent tracers or lubricants comprising the inventive DPPs I. Usually, such lubricant compositions, e.g. for a refrigerant, comprise an oil selected from the group consisting of naphthalenic oils, paraffinic oils, alkylated benzene oils, polyalkyl silicate oils, polyglycols, esters, polyether polyols, polyvinyl ethers, polycarbonates, fluorinated silicones. perfluoroethers, aromatic compounds with fluoroalkyloxy or fluoroalkylthio substituents. The amount of the inventive DPP I in the lubricant is chosen generally in an amount of from 100 to 1000 ppm. If the inventive compound I is water-soluble, it could be used as tracer in water as well.

A particular embodiment of this invention concerns ink jet inks comprising the inventive fluorescent compositions.

The desired ink may contain up to 30 % by weight of the fluorescent composition, but will generally be in the range of 0.1 to 10, preferably from 0.1 to 8% by weight of the total ink composition for most thermal ink jet printing applications.

Further, the inks usually contain polymeric dispersants such as random, block, branched or graft polymers or copolymers. Most preferred are polymeric dispersants made by the group transfer polymerization process, because in general these are free from higher molecular weight species that tend to plug pen nozzles.

In AB or BAB block copolymers, the A segment usually is a hydrophobic homopolymer or copolymer which serves to link with the inventive fluorescent composition and the B block generally is a hydrophilic homopolymer or copolymer, or salts thereof and serves to disperse the pigment in the preferably chosen aqueous medium. Such polymeric dispersants and the synthesis thereof are known from e.g. US 5,085,698.

ABC triblocks are also useful as dispersants. In the ABC triblock, the A block usually is a polymer compatible with water, the B block is a polymer capable of binding to the fluorescent composition and the C block is compatible with the organic solvent. Preferably the A and C blocks are end blocks. ABC triblocks and their synthesis are disclosed e.g. in EP-A 556,649. Suitable graft polymers are disclosed in US 5,231,131.

Representative compounds useful for this purpose include e.g. polymers of polyvinyl alcohol, cellulosics and ethylene oxide modified polymers, and dispersant compounds containing ionisable groups such as acrylic acid, maleic acid or sulfonic acid.

The polymeric dispersant is generally present in an amount in the range of from 0.1 to 30, preferably from 0,1 to 8% by weight of the total ink composition.

In addition to, or in place of the preferred polymeric dispersants, surfactants may be used as dispersants. These may be anionic, nonionic, or amphoteric surfactants. A detailed list of non-polymeric as well as some polymeric dispersants is disclosed in the section on dispersants of Manufacturing Confection Publishing Co., (1990) p. 110-129, McCutcheon's Functional Materials, North America Edition.

Usually the ink contains an aqueous medium such as water or a mixture of water and at least one water-soluble organic solvent. Water-soluble organic solvents are well known, representative examples of which are disclosed in e.g. US 5,085,698. Selection of a suitable mixture of water and water-soluble organic solvent depends on usually requirements of the specific application such as desired surface tension and viscosity, drying time of the ink, and the media substrate onto which the ink will be printed.

Particularly preferred is a mixture of a water-soluble solvent having at least two hydroxyl groups, e.g. diethylene glycol, and water, especially deionized water.

In the event that a mixture of water and a water-soluble organic solvent is used as aqueous medium, water usually would comprise from 30 to 95, preferably 60 to 95% by weight, based on the total weight of the aqueous medium.

The amount of aqueous medium generally is in the range of from 70 to 99.8, preferably from 84 to 99.8%, based on the total weight of the ink.

The ink may contain other ingredients well known to those skilled in the art such as surfactants to alter surface tension as well as to maximize penetration. However, because surfactants may destabilize dispersions, care should be taken to insure compatibility of the surfactant with the other ink components. In general, in aqueous inks, the surfactants may be present in amounts ranging from 0.01 to 5, preferably from 0.2 to 3% by weight, based on the total weight of the ink.

Biocides may be used in the ink compositions to inhibit growth of microorganisms. Sequestering agents such as EDTA may also be included to eliminate deleterious effects of heavy metal impurities. Other known additives, such as viscosity modifiers may also be added.

A further embodiment concerns the use of the inventive fluorescent compounds I in phase change ink jet inks. The preparation of such inks is well known in the art, e.g. described in detail in EP-A 816, 410.

For the pigmentation of high molecular weight organic material the inventive DPPs I, optionally in the form of masterbatches, usually are mixed with the high molecular weight organic materials using roll mills, mixing apparatus or grinding apparatus. Generally, the pigmented material is subsequently brought into the desired final form by conventional processes, such as calandering, compression molding, extrusion, spreading, casting or injection molding. In order to prepare non-rigid moldings or to reduce their brittleness it is often desired to incorporate so-called plasticizers into the high molecular weight organic materials prior to forming. Examples of compounds which can be used as such plasticizers are esters of phosphoric acid, phthalic acid or sebacic acid. The plasticizers can be added before or after the incorporation of the inventive DPPs I into the polymers. It is also possible, in order to achieve different hues, to add fillers or other coloring constituents such as white, color or black pigments in desired amounts to the high molecular weight organic materials in addition to the inventive DPPs I.

For pigmenting lacquers, coating materials and printing inks the high molecular weight organic materials and the inventive DPPs I, alone or together with additives, such as fillers, other pigments, siccatives or plasticizers, are generally dissolved or dispersed in a common organic solvent or solvent mixture. In this case it is possible to adopt a procedure whereby the individual components are dispersed or dissolved individually or else two or more are dispersed or dissolved together and only then are all of the components combined.

The present invention additionally relates to inks comprising a coloristically effective amount of the pigment dispersion of the inventive DPPs I.

Processes for producing inks especially for ink jet printing are generally known and are described for example in US 5,106,412.

The inks can be prepared, for example, by mixing the pigment dispersions comprising the inventive DPPs I with polymeric dispersants.

The mixing of the pigment dispersions with the polymeric dispersant takes place preferably in accordance with generally known methods of mixing, such as stirring or mechanical mixing; it is preferably advisable to use intensive mechanical mixers such as the so-called ULTRATURAX® stirrer from Kunkel & Jahn, Staufen (Germany).

When mixing a DPP I with polymeric dispersants it is preferred to use a water-dilutable organic solvent.

The weight ratio of the pigment dispersion to the ink in general is chosen in the range of from 0.001 to 75% by weight, preferably from 0.01 to 50% by weight, based on the overall weight of the ink.

Examples of suitable polymeric dispersants are carboxyl-containing polyacrylic resins such as polymeric methacrylic or crotonic acids, especially those obtained by addition polymerization of acrylic acid or acrylic acid and other acrylic monomers such as acrylates. Depending on the field of use or when using DPP I, it is also possible, if desired, to admix a small proportion of a water-miscible organic solvent in from 0.01 to 30% by weight, based on the overall weight of the ink, and/or to admix water and/or bases so as to give a pH in the range from 7 to 11. It may likewise be advantageous to add preservatives, antifoams, surfactants, light stabilizers and pH regulators, for example, to the ink of the invention, depending on the field of use.

Examples of suitable pH regulators are inorganic salts such as lithium hydroxide or lithium carbonate, quaternary ammonium hydroxide or ammonium carbonate. Examples of preservatives and antifoams are, for example, sodium dehydroacetate, 2,2-dimethyl-6-acetoxydioxane or ammonium thioglycolate. It is also possible to employ known agents which regulate the viscosity or the surface tension and are described in e.g. US 5,085,698.

Examples of water-miscible organic solvents are aliphatic C₁-C₄alcohols, such as methanol, ethanol, n-propanol, isopropanol, n-butanol, tert.-butanol, ketones such as acetone methyl ethyl ketone, methyl isobutyl ketone or diacetone alcohol, and also polyols, Cellosolves® and carbitols, such as ethylene glycol, diethylene glycol, triethylene glycol, glycerol, propylene gylcol, ethylene glycol monomethyl or monoethyl ether, propylene glycol methyl ether, dipropylene glycol methyl ether, tripropylene glycol methyl ether, ethylene glycol phenyl ether, propylene glycol phenyl ether, diethylene glycol monomethyl or monoethyl ether, diethylene glycol monobutyl ether, triethylene glycol monomethyl or monoethyl ether, and also N-methyl-2-pyrrolidone, 2-pyrrolidone, N,N'-dimethylformamide or N,N'-dimethylacetamide.

If desired, the ink prepared as described above can be worked up further. The working up of the ink can be carried out by the customary methods for working up dispersions, by separation techniques, such as sieving or centrifuging the coarse particles from the resulting dispersion. It has been found advantageous, too, to carry out centrifuging in two stages of different intensity, e.g. centrifuging in a first step for from ten minutes to one hour at from 2000 to 4000 rpm and then, in a second step, for from 10 minutes to one hour at from 6000 to 10000 rpm.

Following centrifuging or sieving, the dispersion usually can be used directly as an ink for ink jet printing, for example.
The present invention additionally relates to a process for producing color filters comprising a transparent substrate and applied thereon a red, blue and green layer in any desired sequence, by using a red compound I and known blue and green compounds. The different colored layers preferably exhibit patterns such that over at least 5% of their respective surface they do not overlap and with very particular preference do not overlap at all.

The preparation and use of color filters or color-pigmented high molecular weight organic materials are well-known in the art and described e.g. in Displays 14/2, 1151 (1993), EP-A 784085, or GB-A 2,310,072.

The color filters can be coated for example using inks, especially printing inks, which can comprise pigment dispersions comprising the inventive DPPs I or can be prepared for example by mixing a pigment dispersion comprising a DPP I with chemically, thermally or photolytically structurable high molecular weight organic material (so-called resist). The subsequent preparation can be carried out, for example, in analogy to EP-A 654 711 by application to a substrate, such as a LCD, subsequent photostructuring and development.

Particular preference for the production of color filters is given to pigment dispersions comprising a DPP I which possess non-aqueous solvents or dispersion media for polymers.

The present invention relates, moreover, to toners comprising a pigment dispersion containing a DPP I or a high molecular weight organic material pigmented with a DPP I in a coloristically effective amount.

In a particular embodiment of the process of the invention, toners, coating materials, inks or colored plastics are prepared by processing masterbatches of toners, coating materials, inks or colored plastics in roll mills, mixing apparatus or grinding apparatus.

The present invention additionally relates to colorants, colored plastics, polymeric ink particles, or non-impact-printing material comprising an inventive DPP I pigment, preferably in the form of a dispersion, or a high molecular weight organic material pigmented with a DPP I in a coloristically effective amount.

A coloristically effective amount of the pigment dispersion according to this invention comprising an inventive DPP I denotes in general from 0.0001 to 99.99% by weight, preferably from 0.001 to 50% by weight and, with particular preference, from 0.01 to 50% by weight, based on the overall weight of the material pigmented therewith.

Further, the inventive compounds I can be used for textile application and for the dying of paper.

In contrast to known red fluorescent dyes (thioindigos) the inventive DPPs I can be applied to color polyamides, because they do not decompose during the incorporation into the polyamides. Further, they exhibit an exceptionally good lightfastness, a superior heat stability, especially in plastics.

### Examples

The solid state absorbance spectra are measured on a Perkin-Elmer Lambda 9 UVNIS-spectrometer and the solid state fluorescence spectra on a Perkin-Elmer MPF 66 with a 5cm Ulbricht-sphere. The measurements are carried out with flexible PVC containing 0.02% by weight of the inventive compounds.

Synthesis Example 1: Sodium hydride (60 % dispersion in mineral oil, 2.56 g, 64 mmol) is added to a slurry of 1,4-diketo-3,6-bis-(4-dimethylaminophenyl)pyrrolo[3,4-c]pyrrole (obtained according to example 1 of EP-A 353,184) (6.0 g, 16.0 mmol) and tetraethyl ammonium bromide (0.344 g, 1.6 mmol) in 1-methyl-2-pyrrolidone (300 ml) at room temperature. After 2 hours, 1-dodecyliodide (19 g, 64 mmol) in 1-methyl-2-pyrrolidone (50 ml) is added over a 10-minutes period. The reaction mixture is then warmed up to 80°C for 24 hours. After cooling to room temperature, water (300 ml) is added to the reaction mixture which is then warmed up to 80°C for one hour. After cooling to 0°C, the supernatant liquid is decanted and the residual solid is crystallized from ethanol. The obtained crystals are washed with a small amount of first ethanol then n-hexane and thereafter dried in an atmosphere under reduced pressure at 50°C for 24 hours. Yield: 7.08 g (62%) of a violet powder, exhibiting a red fluorescence in acetone, chloroform and dimethylsulfoxide ("DMSO").
Elemental analysis: C: 77.64% (calc. 77.70%), H: 9.93% (calc. 9.92%), N: 7.81% (calc. 7.88%)

Synthesis Example 2: Synthesis Example 1 is repeated except that methyl iodide is used as alkylating agent. Yield: 54%, exhibiting a red fluorescence in acetone, chloroform and DMSO.
Elemental analysis: C: 71.63% (calc. 71.62%), H: 6.43% (calc. 6.51%), N: 13.53% (calc. 13.92%)

Example 1: (a) Triphenyl amine (98.32 g. 0.393 mol) is suspended in DMF (280 ml). Phosphorus oxychloride (66.24 g. 0.432 mol) is added dropwise to it over a 30-minutes period without external cooling. After stirring for one more hour, the reaction is heated to 80°C (bath temperature) for 2 ½ hours. After cooling to room temperature, the reaction is slowly poured onto ice-cold water (8 liters) with vigorous stirring. After 30 minutes, aqueous sodium hydroxide (5 N, 250 ml) is added to the reaction, and stirring is continued for one hour. The obtained precipitate is filtered off, washed with water (2 liters), then with methanol (2 litres) and dried to give 4-diphenylaminobenzaldehyde as a beige solid (90.47 g. 0.331 mol, 84 %), which is used in the next step without further purification.

(b) 48.7 g of the above obtained 4-diphenylaminobenzaldehyde (0.178 mol) is suspended in formic acid (400 ml). Hydroxylamine sulfate is added (16.08 g, 0.098 mol), followed by sodium formiate (14.15 g, 0.214 mol). The reaction is heated under reflux for 3 hours. Then the solvent is removed under vacuum. The residue is suspended in toluene (800 ml). The residual solid is filtered off and discarded. Solvents are evaporated and the residue is dissolved in a minimum amount of dichloromethane. This solution is filtered through a pad of silica gel, using dichloromethane as solvent. The solvent is then evaporated under vacuum. The thus obtained solid is taken up in toluene (350 ml) and heated to reflux in the presence of charcoal. After filtering hot, the solvent is removed from the filtrate to give 4-diphenylaminobenzonitrile (42.01 g, 0.155 mol, 87%) as a beige solid, which is taken to the next step without further purification.

(c) Sodium pieces (24.5 g, 1.064 mol) are added to tert.-amyl alcohol (400 ml). Then 20 mg of anhydrous FeCl₃ are added. The reaction mixture is slowly heated until a gentle reflux is obtained. After two hours, all sodium is reacted. 134.54 g of the above obtained 4-diphenylaminobenzonitrile (0.501 mol) are added in portions over a period of 15 minutes. Then di-tert.-butyl succinate (79.5 g, 0.346 mol) in tert.-amyl alcohol (300 ml) is added over 1.75 hours. After an additional hour of heating to reflux, the reaction mixture is cooled to room temperature and stirred overnight. Then, the reaction mixture is slowly added into a mixture of water (1200 ml) and methanol (600 ml) and stirred for 3 hours. The thus obtained solid is then filtered, washed with first water and then ethanol, and afterwards dried at 50°C overnight. 70.96 g (0.114 mol, 46%) of 1,4-diketo-3,6-bis-(4-diphenylaminophenyl)-pyrrolo[3,4-c]pyrrole are obtained as a violet powder. ¹H-NMR (300 MHz, d⁶-DMSO): 6.91 (d, 4H, J = 9 Hz); 7.15-7.22 (m, 12 H); 7.38-7.43 (m, 8 H); 8.32 (d, 4 H, J = 9 Hz); 11.02 (broad s, 2 H).

(d) Synthesis Example 2 is repeated except that the above obtained 1,4-diketo-3,6-bis-(4-diphenylaminophenyl)pyrrolo[3,4-c]pyrrol is used. Yield: 56%, exhibiting a red fluorescence in acetone, chloroform and DMSO.
Elemental analysis: C: 80.76% (calc. 81.21%), H: 5.30% (calc. 5.27%), N: 8.82% (calc, 8.61 %), max. absorbance (solid state): 555 nm; max. fluorescence (solid state): 607 nm.

Synthesis Example 3: 2.09 g (4.75 mmol) 1,4-diketo-3,6-bis-(4-biphenyl)-pyrrolo-(3,4-c)-pyrrole are slurred in 30 ml of 1-methyl-2-pyrrrolidinone for 2 hours at room temperature. 1.29 g (11.52 mmol) of potassium tert.-butoxide is added to the slurry under nitrogen. After stirring for 2 hours, 2.05 g (11.1 mmol) of 3-methylbenzyl bromide is added to the reaction mixture and then the mixture is stirred additionally for 2 hours. The mixture is poured into 50 ml of water and the red solid is filtered off and purified by column chromatography ( silica gel, dichloromethane as eluent). After drying, 1.89 g (61%) of a red solid are obtained.

Synthesis Example 4: example 20 is repeated except that 3,5-dimethylbenzyl bromide is used as alkylating agent. Red solid (yield: 24%).

Example 2: (a) 24.6 g of potassium t-butoxide, 30 g of 2- naphthonitrile and 200 ml of tert.-amyl alcohol are heated up to 100°C under a nitrogen atmosphere. As soon as the this temperature is reached, a solution of 23 g of di-n-butyl succinate and 70 ml of tert.-amyl alcohol is added over one hour using a dropping funnel. When the addition is complete, the reaction mixture is kept for 16 hours at 100°C, then it is cooled to 65°C, afterwards neutralized with 20 ml of glacial acetic acid and boiled briefly under reflux. The resultant pigment suspension is filtered at room temperature. The obtained filter cake is suspended in 300 ml of methanol and the pigment is isolated by filtration, then finally washed with methanol and water until washings run colorless, thereafter dried at 100°C in vacuo. Affording 26.1 g (69%, based on dibutyl succinate) of pure 1,4-diketo-3,6-bis-(2-naphthyl)-pyrrolo-(3,4-c)-pyrrole.

(b) Synthesis Example 4 is repeated except that 1,4-diketo-3,6-bis-(2-naphthyl)-pyrrolo-(3,4-c)-pyrrole is used as starting material. Red solid (yield: 36%).

Example 3: example 2 is repeated except that benzyl bromide is used as alkylating agent. Orange solid (yield: 30 %).

Example 4: example 2 is repeated except that 2-methylbenzyl bromide is used as alkylating agent. Orange solid (yield: 30 %).

Example 5: example 2 is repeated except that 2-phenylbenzyl bromide is used as alkylating agent. Red solid (yield: 8 %).

Synthesis Example 5: 2.0 g (4.54 mmol) 1,4-diketo-3,6-bis-(4-biphenyl)-pyrrolo-(3,4-c)-pyrrole are slurred in 30 ml of 1-methyl-2-pyrrolidinone for two hours at room temperature. 1.3 g (11.61 mmol) of potassium tert.-butoxide are added to the slurry under nitrogen. After stirring for two hours, 2.07 g (11.2 mmol) of 2-methylbenzyl bromide are added to the reaction mixture and then the mixture is stirred additionally for two hours. The mixture is poured into 50 ml of water and the red solid is filtered off and purified by column chromatography (silica gel, dichloromethane as eluent). After drying, 0.866 g (29%) of a red solid are obtained.

Example 6: Synthesis example 5 is repeated except that 3-methylbenzyl bromide and 1,4-diketo-3,6-bis-(2-naphthyl)-pyrrolo-(3,4-c)-pyrrole are used as alkylating agent and starting material, respectively. Red solid (yield: 30%).

Example 7: example 6 is repeated except that 4-methylbenzyl bromide is used as alkylating agent. Red solid (yield: 36%).

Example 8: example 6 is repeated except that 4-phenylbenzyl bromide is used as alkylating agent. Orange solid (yield: 30 %).

Synthesis Example 6: 2.2 g (5.0 mmol) 1,4-diketo-3,6-bis-(4-biphenyl)-pyrrolo-(3,4-c)-pyrrole are slurred in 20 ml of 1-methyl-2-pyrrrolidinone for two hours at room temperature. 1.4 g (13.0 mmol) of potassium tert.-butoxide are added to the above obtained slurry under nitrogen. After stirring for two hours, 2.78 g (12 mmol) of 2-iodoethyl benzene are added to the reaction mixture. Then, the mixture is heated up to 80°C and stirred additionally for three hours. After cooling to room temperature, the mixture is poured into 50 ml of water and the obtained red solid is filtered off and purified by column chromatography (silica gel, dichloromethane as eluent). After drying, 0.16 g (5 %) of a red solid are obtained.

Example 9: Synthesis example 6 is repeated except that 1,4-diketo-3,6-bis-(2-naphthyl)-pyrrolo-(3,4-c)-pyrrole is used as starting material (yield: 29 %).

Example 10: example 2 is repeated except that 3-phenylbenzyl bromide is used as alkylating agent. Orange solid (yield: 35 %).

Example 11: example 2 is repeated except that 3-methoxybenzyl bromide is used as alkylating agent. Orange solid (yield: 30 %).

Example 12: To the below mentioned engineering plastics (each 400 g) in chip form the inventive compounds (each 0.12 g) are added in a paint shaker and shaken there for 90 seconds. Thereafter, the thus obtained chips comprising the adhered inventive compounds are molded at the temperatures specified in Table 1 below using a BA400 Battenfeld injection molder.

**Table 1**

| Engineering plastic | Temperature settings (°C) | | |
|---|---|---|---|
| | Nozzle | Middle | Rear |
| HIPS | 232 (450°F) | 232 (450°F) | 232 (450°F) |
| HIPS : high impact polystyrene (825P1 from Fina Oil and Chemical; melt flow (g/10 min): 8 (reference ASTM TEST 200/5.0 D-1238)) | | | |

Five chips (from the same series) obtained from the injection molder are collected after the color is distributed homogeneously. The color chips are then mounted, with the thickest part of the chip exposed (0.31 cm (0.122 inches)), in a Cl35A Atlas Xenon Weather-O-Meter. The parameters of the weather-o-meter are listed in Table 2.

The color chips are then exposed to the weather-o-meter for 100, 250, 500, 750, and 1000 hours. The color chips are rated after each fading interval is reached. The lightfastness is subjectively evaluated using a gray scale rating of 1-5. A rating of 5 indicates no fade or color difference. In cases where the color chip appears to darken a rating of d for darkening is applied to the gray scale rating. In cases where the color chip loses most of its color a rating of f for fading is applied to the gray scale rating.

**Table 2:**

| Parameters for the fading test | |
|---|---|
| Automatic voltage 0.35 W/m² at 340 nm | |
| | Light Cycle Settings |
| | Temperature [°C] |
| Black Panel¹⁾ | 63 |
| Wet Bulb Depression²⁾ | 10 |
| Conditioning Water³⁾ | 30 |
| automatic voltage: controls irradiance level (similar to the average irradiance found on a clear summer day in southern Florida) | |

| | |
|---|---|
| 1) temperature is measured by a sensor attached to the specimen holder; provides a temperature reading resulting from the chamber air and any heating due to the xenon light | |
| 2) difference between air temperature (measured by the so-called dry bulb sensor) and wet bulb temperature (measured by wet bulb sensor, whereby the wet bulb is covered by a moistened wick; due to the cooling effect of evaporation, the wet bulb reading is usually lower than the dry bulb reading (except at 100% relative humidity). | |

**Table 3:**

| HIPS evaluation | | | | | |
|---|---|---|---|---|---|
| used DPP of example no. | Lightfastness evaluation using the gray scale | | | | |
| | 100 h | 250 h | 500 h | 700 h | 1000 h |
| 1d | 4/5 | 4 | 4d | 3d | 3d |
| Vat Red 41 | 3 | 2/3 | 1f | | |

Synthesis Example 7: 2.09 g (4.75 mmol) 1,4-diketo-3,6-bis-(4-biphenyl)-pyrrolo-(3,4-c)-pyrrole are slurred in 30 ml of 1-methyl-2-pyrrolidinone for 2 hours at room temperature. 1.29 g (11.52 mmol) of potassium tert.-butoxide are added to the slurry under an atmosphere of nitrogen. After stirring for 1 h, 1.21 g (10 mmol) of allyl bromide are added to the reaction mixture, and then the mixture is stirred for another 2 hours. The mixture is then poured into 50 ml of water and the red solid is filtered off and purified by column chromatography (silica gel, dichloromethane as eluent). After drying, 1.13 g (60%) of a red solid are obtained.

Example 13: A mixture of 45 g (0.4 mol) of potassium tert.-butoxide, 82 g (0.373 mol) of 9-ethyl-3-cyanocarbazole and 300 ml of tert.-amyl alcohol is heated up to 100°C under a nitrogen atmosphere. As soon as the temperature is reached, a solution of 43 g (0.18 mol) of di-n-butyl succinate and 100 ml of tert.-amyl alcohol are added during 1.5 hours using a dropping funnel. When the addition is complete, the reaction mixture is kept for 16 hours at 110°C, then cooled to 65°C, neutralized with 40 ml of glacial acetic acid and boiled briefly to reflux temperature. The resultant pigment suspension is filtered at room temperature. The filter cake is suspended in 300 ml of methanol and the pigment is isolated by filtration, then finally washed with methanol and water until washings run colorless, then dried at 100°C under an atmosphere of reduced pressure. 10.5 g (11%) of pure 1,4-diketo-3,6-bis-(3-(9-ethylcarbazole))-pyrrolo-(3,4-c)-pyrrole are obtained.

Example 14: Synthesis Example 7 is repeated except that 1,4-diketo-3,6-bis-(2-naphthyl)-pyrrolo-(3,4-c)pyrrole and 3,5-di-tert.-butylbenzylbromide are used. A red solid (36%) is obtained.

Example 15: Example 14 is repeated except that 3,5-dimethylbenzyl bromide is used instead of 3,5-di-tert.-butylbenzylbromide. A red solid (30%) is obtained.

Example 16: Example 15 is repeated except that 1-(4-cyanophenyl)-2-(3,5-di-tert.-butylphenyl)-trans-ethylene is used instead of 9-ethyl-3-cyanocarbazole. A red solid (5%) is obtained.

Example 17: Example 16 is repeated except that 3,5-dimethylbenzyl bromide is used as alkylating agent. A red solid (8%) is obtained.

## Claims

1. Fluorescent diketopyrrolopyrroles ("DPPs") of the formula I 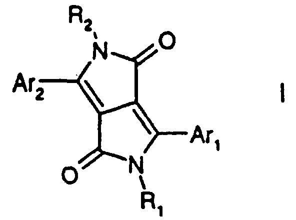 wherein R₁ and R₂, independently from each other, stand for C₁-C₂₅-alkyl, allyl which can be substituted one to three times with C₁-C₃alkyl or Ar₃, -CR₃R₄-(CH₂)ₘ-Ar₃,
wherein R₃ and R₄ independently from each other stand for hydrogen or C₁-C₄alkyl, or phenyl which can be substituted one to three times with C₁-C₃ alkyl,
Ar₃ stands for phenyl or 1- or 2-naphthyl which can be substituted one to three times with C₁-C₈alkyl, C₁-C₈alkoxy, halogen or phenyl, which can be substituted with C₁-C₈alkyl or C₁-C₈alkoxy one to three times, and m stands for 0, 1, 2, 3 or 4, and wherein C₁-C₂₅-alkyl or -CR₃R₄-(CH₂)ₘ-Ar₃, preferably C₁-C₂₅-alkyl, can be substituted with a functional group capable of increasing the solubility in water which is selected from a tertiary amino group, -SO₃⁻, or PO₄²⁻,
Ar₁ and Ar₂, independently from each other, stand for 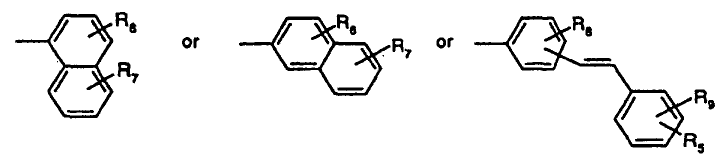 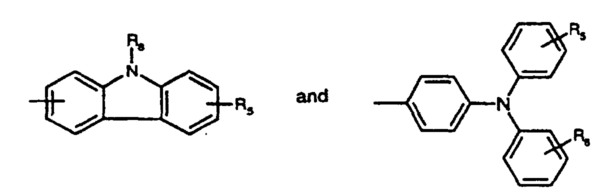 wherein
R₅ stands for C₁-C₆alkyl, -NR₈R₉, -OR₁₀, -S(O)ₙR₈, -Se(O)ₙR₈, or phenyl, which can be substituted one to three times with C₁-C₈alkyl or C₁-C₈alkoxy,
wherein R₈ and R₉, independently from each other, stand for hydrogen, C₁-C₂₅-alkyl, C₅-C₁₂-cycloalkyl, -CR₃R₄-(CH + ₂)ₘ-Ph, R₁₀, wherein R₁₀ stands for C₆-C₂₄-aryl, or a saturated or unsaturated heterocyclic radical comprising five to seven ring atoms, wherein the ring consists of carbon atoms and one to three hetero atoms selected from the group consisting of nitrogen, oxygen and sulfur, wherein Ph, the aryl and heterocyclic radical can be substituted one to three times with C₁-C₈alkyl, C₁-C₈alkoxy, or halogen, or R₈ and R₉ stand for -C(O)R₁₁, wherein R₁₁ can be C₁-C₂₅-alkyl, C₅-C₁₂-cycloalkyl, R₁₀, -OR₁₂ or -NR₁₃R₁₄, wherein R₁₂, R₁₃, and R₁₄ stand for C₁-C₂₅-alkyl, C₂-C₁₂-cycloalkyl, C₆-C₁₄-aryl, or
a saturated or unsaturated heterocyclic radical comprising five to seven ring atoms,
wherein the ring consists of carbon atoms and one to three hetero atoms selected from the group consisting of nitrogen, oxygen and sulfur, wherein the aryl and heterocyclic radical can be substituted one to three times with C₁-C₈alkyl or C₁-C₈alkoxy, or -NR₈R₉ stands for a five- or sixmembered heterocyclic radical in which R₈ and R₉ together stand for tetramethylene, pentamethylene, -CH₂-CH₂-O-CH₂-CH₂-, or -CH₂-CH₂NR₅-CH₂-CH₂-, preferably -CH₂-CH₂-O-CH₂-CH₂-, and n stands for 0, 1, 2 or 3,
and wherein R₆ and R₇, independently from each other, stand for hydrogen or R₅, but do not stand simultaneously for hydrogen, preferably R₆ stands for R₅ and R₇ for hydrogen.

2. Fluorescent diketopyrrolopyrroles according to claim 1, wherein R₁ = R₂ = C₁-C₈alkyl, Ar₁ = Ar₂ = stilbene, and R₈ = R₉ = C₁-C₈alkyl or phenyl.

3. Fluorescent diketopyrrolopyrroles according to claim 1, wherein R₁ = R₂ = C₁-C₈alkyl, -(CH₂)ₘ-Ph, Ar₁ = Ar₂ = stilbene, and R₈ = C₁-C₈alkyl, phenyl or a heterocyclic radical, both unsubstituted or substituted, or C₅-C₁₂-cycloalkyl.

4. Fluorescent diketopyrrolopyrroles: 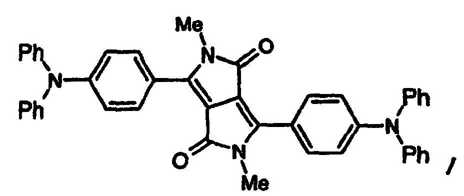 or 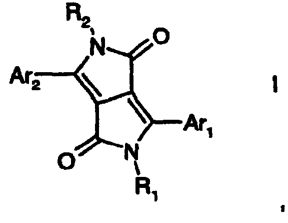 wherein Ar₁ and Ar₂ are 2-naphthyl and
R₁ and R₂ are 3,5-dimethylbenzyl,
R₁ and R₂ are benzyl,
R₁ and R₂ are 2-methylbenzyl,
R₁ and R₂ are 2-phenylbenzyl,
R₁ and R₂ are 3-methylbenzyl,
R₁ and R₂ are 4-methylbenzyl,
R₁ and R₂ are 4-phenylbenzyl,
R₁ and R₂ are 2-phenylethyl,
R₁ and R₂ are 3-phenylbenzyl,
R₁ and R₂ are 3-methoxybenzyl, or
R₁ and R₂ are 3,5-di-tert.-butylbenzyl.

5. Fluorescent diketopyrrolopyrroles according to claim 1, 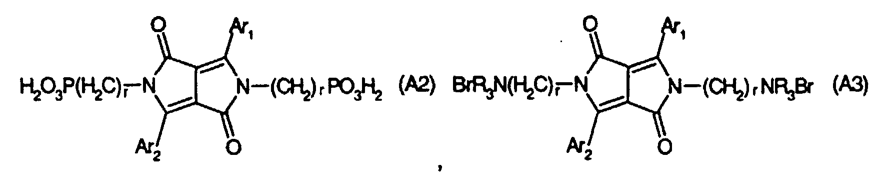 or 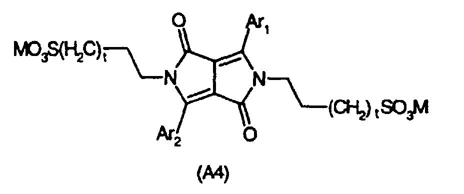 wherein Ar₁ and Ar₂ are as defined in claim 1, r stands for an integer from 2 to 25; instead of linear alkyl groups, wherein the group -(CH₂)ᵣ- could be replaced by branched alkyl groups or aralkyl groups, such as -(CH₂)ᵣ₁-aryl-(CH₂)ᵣ₂-, r₁ and r₂ being whole numbers in the range of from 0 to 10; wherein M stands for a metal ion such as sodium or potassium, and t is 1 or 2.

6. Process for the preparation of a compound I according to claim 1 in treating in a first step the DPP derivative of formula V 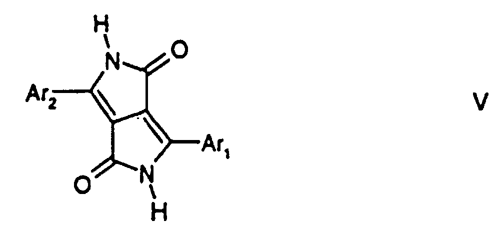 wherein Ar₁ and Ar₂ are defined as in claim 1, with a base, then, in a second step, treating the reaction mixture obtained in the first step with an alkylating agent, wherein in the first step the base is a hydride, an alkali metal alkoxide or a carbonate, and the alkylating agent is a sulfonate, tosylate, mesylate, carbonate, sulfate, or halogen compound of the formula (R₁)_{1 or 2}X, wherein X stands for SO₃-, (p-Me-phenyl)SO₂-, (2,4,6-trimethyl-phenyl)SO₂-, -CO₃-, -SO₄-, or halogen, or a mixture of (R₁)_{1 or 2}X and (R₂)_{1 or 2}X wherein R¹ and R² are defined as in claim 1.

7. Process for the preparation of the compounds I according to claim 1
(a) in treating in a first step the DPP derivative of formula VI 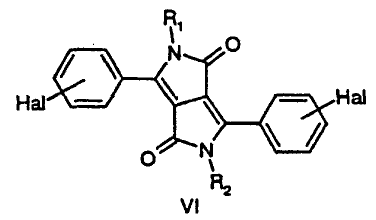 wherein R₁ and R₂ are hydrogen or are defined as in claim 1, Hal stands for halogen, with a nucleophilic agent such as a secondary amine, HNR₈R₉, a thiol, HSR₈, or HS(O)ₙR₈, an alcohol, HOR₁₀, a diselenide, R₈(O)ₙSe-Se(O)ₙR₈, preferably in a molar ratio of DPP VI:nucleophilic agent in the range of 1.2:1 to 0.8:1, or, if R₂ has the same meaning as R₁ in the range of from 1:2.5 to 1:1, in the presence of an anhydrous dipolar aprotic solvent, and of an anhydrous base in an amount in the range of from 0.1 to 15 moles per mole of the nucleophilic agent, at a temperature in the range of from 100 to 220°C and under a pressure in the range of from 100 to 300 kPa, and optionally isolating the obtained compound V,
(b) then treating the obtained compound V (as defined in claim 6), with a base, thereafter in a second step, treating the reaction mixture obtained in the first step of (b) with an alkylating agent, wherein in the first step of (b) the base is a hydride, an alkali metal alkoxide or a carbonate, and the alkylating agent is a sulfonate, tosylate, mesylate, carbonate, sulfate, or halogen compound of the formula (R₁)_{1 or 2}X, wherein X stands for SO₃-, (p-Me-phenyl)-SO₂-, (2,4,6-trimethyl-phenyl)SO₂-, -CO₃-, -SO₄-, or halogen, or a mixture of (R₁)_{1 or 2}X and (R₂)_{1 or 2}X.

8. Method of coloring high molecular weight organic materials by incorporating the DPP compounds I according to any of claims 1 to 4 by known methods in the art.

9. Composition comprising
(a) 0.01 to 50% by weight, based on the total weight of the colored high molecular weight organic material, of a fluorescent DPP I according to any of claims 1 to 4, and
(b) 99.99 to 50% by weight, based on the total weight of the colored high molecular weight organic material, of a high molecular organic material, and
(c) 0 to 50% by weight customary additives based on the total weight of (a) and (b).

10. Composition according to claim 9, wherein the high molecular weight organic material is a polyamide, a polystyrene, preferably high impact polystyrene, polymethylmethacrylate or an ABS copolymer.

11. Use of DPPs I according to any of claims 1 to 5 for the preparation of inks, colorants, pigmented plastics for coatings, non-impact-printing material, color filters, cosmetics, or for the preparation of polymeric ink particles, toners, dye lasers and electroluminescent devices.

## Patentansprüche

1. Fluoreszierende Diketopyrrolopyrrole ("DPPs") der Formel I 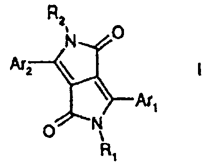 worin R₁ und R₂ unabhängig voneinander für C₁-C₂₅-Alkyl, Allyl, das mit C₁-C₃-Alkyl oder Ar₃ ein- bis dreifach substituiert sein kann, -CR₃R₄-(CH₂)ₘ-Ar₃, worin R₃ und R₄ unabhängig voneinander für Wasserstoff oder C₁-C₄-Alkyl stehen, oder Phenyl, das mit C₁-C₃-Alkyl ein- bis dreifach substituiert sein kann, stehen,
Ar₃ für Phenyl oder 1- oder 2-Naphthyl steht, das mit C₁-C₈-Alkyl, C₁-C₈-Alkoxy, Halogen oder Phenyl, welches mit C₁-C₈-Alkyl oder C₁-C₈-Alkoxy ein- bis dreifach substituiert sein kann, ein- bis dreifach substituiert sein kann und m für 0, 1, 2, 3 oder 4 steht, und worin C₁-C₂₅-Alkyl oder -CR₃R₄-(CH₂)ₘ-Ar₃, vorzugsweise C₁-C₂₅-Alkyl, mit einer funktionellen Gruppe substituiert sein kann, die die Löslichkeit in Wasser erhöhen kann, welche aus einer tertiären Aminogruppe, -SO₃⁻ oder PO₄²⁻ ausgewählt ist,
Ar₁ und Ar₂ unabhängig voneinander für 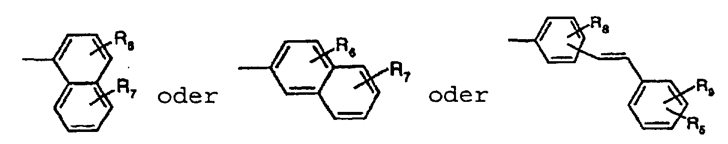 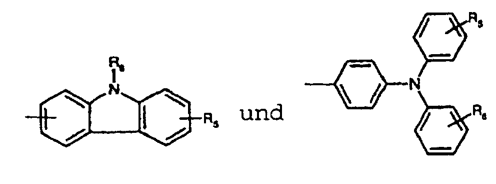 stehen, worin
R₅ für C₁-C₆-Alkyl, -NR₈R₉, -OR₁₀, -S(O)ₙR₈, -Se(O)ₙR₈ oder Phenyl steht, das mit C₁-C₈-Alkyl oder C₁-C₈-Alkoxy einbis dreifach substituiert sein kann,
worin R₈ und R₉ unabhängig voneinander für Wasserstoff, C₁-C₂₅-Alkyl, C₅-C₁₂-Cycloalkyl, -CR₃R₄-(CH₂)ₘ-Ph, R₁₀, wobei R₁₀ für C₆-C₂₄-Aryl steht, oder einen gesättigten oder ungesättigten heterocyclischen Rest, umfassend fünf bis sieben Ringatome, stehen, wobei der Ring aus Kohlenstoffatomen und ein- bis drei Heteroatomen, ausgewählt aus der Gruppe, bestehend aus Stickstoff, Sauerstoff und Schwefel, besteht, wobei Ph, der Aryl- und heterocyclische Rest mit C₁-C₈-Alkyl, C₁-C₈-Alkoxy oder Halogen ein- bis dreifach substituiert sein können, oder R₈ und R₉ für -C(O)R₁₁ stehen, worin R₁₁ C₁-C₂₅-Alkyl, C₅-C₁₂-Cycloalkyl, R₁₀, -OR₁₂ oder -NR₁₃R₁₄ sein kann, worin R₁₂, R₁₃ und R₁₄ für C₁-C₂₅-Alkyl, C₅-C₁₂-Cycloalkyl, C₆-C₂₄-Aryl, oder
einen gesättigten oder ungesättigten heterocyclischen Rest, umfassend fünf bis sieben Ringatome, stehen, wobei der Ring aus Kohlenstoffatomen und ein bis drei Heteroatomen, ausgewählt aus der Gruppe, bestehend aus Stickstoff, Sauerstoff und Schwefel, besteht, wobei der Aryl- und heterocyclische Rest mit C₁-C₈-Alkyl oder C₁-C₈-Alkoxy einbis dreifach substituiert sein kann, oder -NR₈R₉ für einen fünf- bis sechsgliedrigen heterocyclischen Rest steht, worin R₈ und R₉ zusammen für Tetramethylen, Pentamethylen, -CH₂-CH₂-O-CH₂-CH₂- oder -CH₂-CH₂-NR₅-CH₂-CH₂-, vorzugsweise -CH₂-CH₂-O-CH₂-CH₂-, stehen, und n für 0, 1, 2 oder 3 steht, und worin R₆ und R₇ unabhängig voneinander für Wasserstoff oder R₅ stehen, jedoch nicht gleichzeitig für Wasserstoff stehen, vorzugsweise steht R₆ für R₅ und R₇ steht für Wasserstoff.

2. Fluoreszierende Diketopyrrolopyrrole nach Anspruch 1, worin R₁ = R₂ = C₁-C₈-Alkyl, Ar₁ = Ar₂ = Stilben und R₈ = R₉ = C₁-C₈-Alkyl oder Phenyl.

3. Fluoreszierende Diketopyrrolopyrrole nach Anspruch 1, worin R₁ = R₂ = C₁-C₈-Alkyl, -(CH₂)ₘ-Ph, Ar₁ = Ar₂ = Stilben, und R₈ = C₁-C₈-Alkyl, Phenyl oder ein heterocyclischer Rest, sowohl unsubstituiert als auch substituiert, oder C₅-C₁₂-Cycloalkyl.

4. Fluoreszierende Diketopyrrolopyrrole: 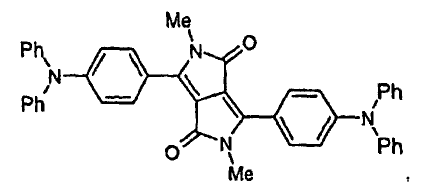 oder 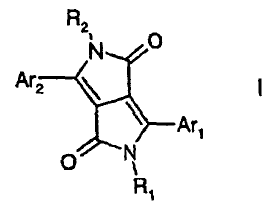 worin Ar₁ und Ar₂ 2-Naphthyl darstellen und
R₁ und R₂ 3,5-Dimethylbenzyl darstellen,
R₁ und R₂ Benzyl darstellen,
R₁ und R₂ 2-Methylbenzyl darstellen,
R₁ und R₂ 2-Phenylbenzyl darstellen,
R₁ und R₂ 3-Methylbenzyl darstellen,
R₁ und R₂ 4-Methylbenzyl darstellen,
R₁ und R₂ 4-Phenylbenzyl darstellen,
R₁ und R₂ 2-Phenylethyl darstellen,
R₁ und R₂ 3-Phenylbenzyl darstellen,
R₁ und R₂ 3-Methoxybenzyl darstellen oder
R₁ und R₂ 3,5-Di-tert-butylbenzyl darstellen.

5. Fluoreszierende Diketopyrrolopyrrole nach Anspruch 1 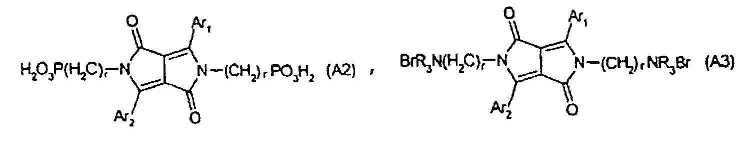 oder 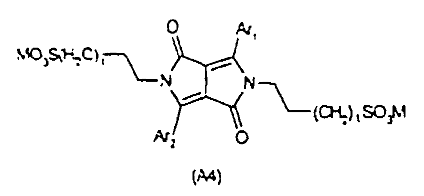 worin Ar₁ und Ar₂ wie in Anspruch 1 definiert sind, r für eine ganze Zahl von 2 bis 25 steht;
wobei die Gruppe -(CH₂)ᵣ-, anstelle von linearen Alkylgruppen, durch verzweigte Alkylgruppen oder Aralkylgruppen ersetzt sein könnte, wie -(CH₂)ᵣ₁-Aryl-(CH₂)ᵣ₂-, r₁ und r₂ ganze Zahlen im Bereich von 0 bis 10 sind, M für ein Metallion, wie Natrium oder Kalium, steht und t 1 oder 2 ist.

6. Verfahren zur Herstellung von Verbindungen I nach Anspruch 1 beim Behandeln in einem ersten Schritt des DPP-Derivats der Formel V 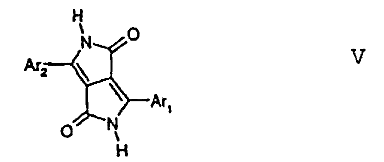 worin Ar₁ und Ar₂ wie in Anspruch 1 definiert sind, mit einer Base, dann, in einem zweiten Schritt Behandeln des in dem ersten Schritt erhaltenen Reaktionsgemisches mit einem Alkylierungsmittel, wobei in dem ersten Schritt die Base ein Hydrid, ein Alkalimetallalkoxid oder ein Carbonat darstellt, und das Alkylierungsmittel eine Sulfonat-, Tosylat-, Mesylat-, Carbonat-, Sulfat- oder Halogenverbindung der Formel (R₁)₁ oder ₂X, worin X für SO₃-, (p-Me-Phenyl)SO₂-, (2,4,6-Trimethyl-phenyl)SO₂-, -CO₃-, -SO₄oder Halogen steht oder ein Gemisch von (R₁)₁ oder ₂X und (R₂)_{1 oder 2}X darstellt, wobei R¹ und R² wie in Anspruch 1 definiert sind.

7. Verfahren zur Herstellung der Verbindungen I nach Anspruch 1:
(a) beim Behandeln in einem ersten Schritt des DPP-Derivats der Formel VI 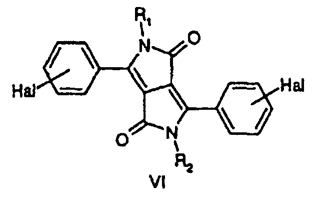 worin R₁ und R₂ Wasserstoff darstellen oder wie in Anspruch 1 definiert sind, Hal für Halogen steht, mit einem nucleophilen Mittel, wie einem sekundären Amin, HNR₈R₉, einem Thiol, HSR₈ oder HS(O)ₙR₈, einem Alkohol, HOR₁₀, einem Diselenid, R₈(O)ₙSe-Se(O)ₙR₈, vorzugsweise in einem Molverhältnis von DPP VI:nucleophilem Mittel im Bereich von 1,2:1 bis 0,8:1, oder, wenn R₂ die gleiche Bedeutung wie R₁ aufweist, im Bereich von 1:2,5 bis 1:1, in Gegenwart eines wasserfreien dipolaren aprotischen Lösungsmittels und einer wasserfreien Base, in einer Menge im Bereich von 0,1 bis 15 Mol pro Mol des nucleophilen Mittels, bei einer Temperatur im Bereich von 100 bis 220°C und unter einem Druck im Bereich von 100 bis 300 kPa, und gegebenenfalls Isolieren der erhaltenen Verbindung V,
(b) dann Behandeln der erhaltenen Verbindung V (wie in Anspruch 2 definiert) mit einer Base, anschließend, in einem zweiten Schritt, Behandeln des in dem ersten Schritt von (b) erhaltenen Reaktionsgemisches mit einem Alkylierungsmittel, worin in dem ersten Schritt von (b) die Base ein Hydrid, ein Alkalimetallalkoxid oder ein Carbonat darstellt, und das Alkylierungsmittel eine Sulfonat-, Tosylat-, Mesylat-, Carbonat-, Sulfat- oder Halogenverbindung der Formel (R₁)_{1 oder 2}X,
worin X für SO₃-, (p-Me-Phenyl)SO₂-, (2,4,6-Trimethylphenyl)SO₂-, -CO₃-, -SO₄- oder Halogen steht oder ein Gemisch von (R₁)_{1 oder 2}X und (R₂)_{1 oder 2}X darstellt.

8. Verfahren zum Färben von organischen Materialien mit hohem Molekulargewicht durch Einarbeiten der DPP-Verbindungen I gemäß einem der Ansprüche 1 bis 4 durch auf dem Fachgebiet bekannte Verfahren.

9. Zusammensetzung, umfassend
(a) 0,01 bis 50 Gewichtsprozent, bezogen auf das Gesamtgewicht des gefärbten organischen Materials mit hohem Molekulargewicht, eines fluoreszierenden DPP I nach einem der Ansprüche 1 bis 4, und
(b) 99,99 bis 50 Gewichtsprozent, bezogen auf das Gesamtgewicht des gefärbten organischen Materials mit hohem Molekulargewicht, eines organischen Materials mit hohem Molekulargewicht, und
(c) 0 bis 50 Gewichtsprozent übliche Additive, die auf dem Gesamtgewicht von (a) und (b) basieren.

10. Zusammensetzung nach Anspruch 9, worin das organische Material mit hohem Molekulargewicht ein Polyamid, ein Polystyrol, vorzugsweise Polystyrol mit hoher Schlagfestigkeit, Polymethylmethacrylat oder ein ABS-Copolymer ist.

11. Verwendung von DPP I nach einem der Ansprüche 1 bis 5 zur Herstellung von Druckfarben, Färbemitteln, pigmentierten Kunststoffen für Beschichtungen, Material für anschlagfreien Druck, Farbfiltern, Kosmetika, oder für die Herstellung von polymeren Druckfarbenteilchen, Tonern, Farblasern und elektrolumineszierenden Vorrichtungen.

## Revendications

1. Dicétopyrrolopyrroles ("DPP") fluorescents de formule I 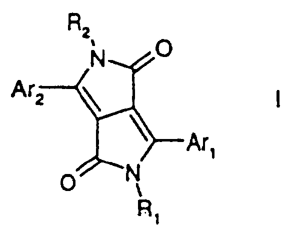 dans laquelle
R₁ et R₂ représentent chacun, indépendamment de l'autre, un groupe alkyle en C₁-C₂₅, allyle qui peut être substitué une à trois fois par un groupe alkyle en C₁-C₃ ou Ar₃, -CR₃R₄-(CH₂)ₘ-Ar₃ où R₃ et R₄ représentent chacun, indépendamment de l'autre, l'hydrogène ou un groupe alkyle en C₁-C₄, ou phényle qui peut être substitué une à trois fois par un groupe alkyle en C₁-C₃,
Ar₃ représente un groupe phényle ou 1- ou 2-naphtyle qui peut être substitué une à trois fois par un groupe alkyle en C₁-C₈, alcoxy en C₁-C₈, halogéno ou phényle qui peut être substitué une à trois fois par un groupe alkyle en C₁-C₈ ou alcoxy en C₁-C₈, et m représente 0, 1, 2, 3 ou 4, et où un groupe alkyle en C₁-C₂₅ ou -CR₃R₄-(CH₂)ₘ-Ar₃, de préférence alkyle en C₁-C₂₅, peut être substitué par un groupe fonctionnel capable d'augmenter la solubilité dans l'eau, qui est choisi parmi un groupe amino tertiaire, -SO₃⁻, ou PO₄²⁻ ,
Ar₁ et Ar₂ représentent chacun, indépendamment de l'autre, 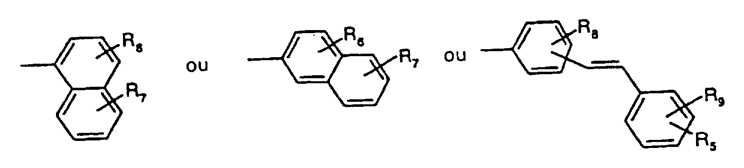 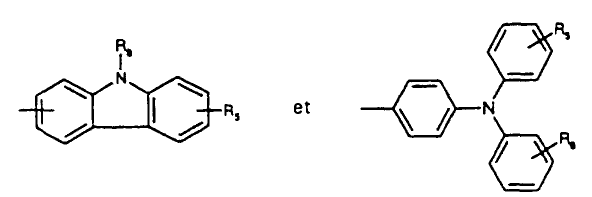 où
R₅ représente un groupe alkyle en C₁-C₆, -NR₈R₉, -OR₁₀, -S(O)ₙR₈, -Se(O)ₙR₈ ou phényle qui peut être substitué une à trois fois par un groupe alkyle en C₁-C₈ ou alcoxy en C₁-C₈,
R₈ et R₉ représentent chacun, indépendamment de l'autre, l'hydrogène, un groupe alkyle en C₁-C₂₅, cycloalkyle en C₅-C₁₂, -CR₃R₄-(CH₂)ₘ-Ph, R_{10,} où R₁₀ représente un groupe aryle en C₆-C₂₄, ou un radical hétérocyclique saturé ou insaturé comprenant cinq à sept atomes cycliques, dont le cycle est constitué d'atomes de carbone et de un à trois hétéroatomes choisis dans la classe formée par l'azote, l'oxygène et le soufre, où Ph, le groupe aryle et le radical hétérocyclique peuvent être substitués une à trois fois par un groupe alkyle en C₁-C₈, alcoxy en C₁-C₈ ou halogèno, ou bien R₈ et R₉ représentent -C(O)R₁₁, où R₁₁ peut être un groupe alkyle en C₁-C₂₅, cycloalkyle en C₅-C₁₂, R₁₀, -OR₁₂ ou -NR₁₃R₁₄, où R_{12,} R₁₃ et R₁₄ représentent chacun un groupe alkyle en C₁-C₂₅, cycloalkyle en C₅-C₁₂, aryle en C₆-C₂₄,
ou
un radical hétérocyclique saturé ou insaturé comprenant cinq à sept atomes cycliques, dont le cycle est constitué d'atomes de carbone et de un à trois hétéroatomes choisis dans la classe formée par l'azote, l'oxygène et le soufre,
où le groupe aryle et le radical hétérocyclique peuvent être substitués une à trois fois par un groupe alkyle en C₁-C₈ ou alcoxy en C₁-C₈,
ou bien -NR₈R₉ représente un radical hétérocyclique penta- ou hexagonal dans lequel R₈ et R₉ représentent ensemble un groupe tétraméthylène, pentaméthylène, -CH₂-CH₂-O-CH₂-CH₂-, ou -CH₂-CH₂-NR₅-CH₂-CH₂-, de préférence -CH₂-CH₂-O-CH₂-CH₂-, et n représente 0, 1, 2 ou 3, et
R₆ et R₇ représentent chacun, indépendamment de l'autre, l'hydrogène ou R₅ mais ne représentent pas simultanément l'hydrogène, de préférence R₆ représente R₅ et R₇ représente l'hydrogène.

2. Dicétopyrrolopyrroles fluorescents selon la revendication 1, dans lesquels R₁ = R₂ = alkyle en C₁-C₈, Ar₁ = Ar₂ = stilbène, et R₈ = R₉ = alkyle en C₁-C₈ ou phényle.

3. Dicétopyrrolopyrroles fluorescents selon la revendication 1, dans lesquels R₁ = R₂ = alkyle en C₁-C₈, -(CH₂)ₘ-Ph, Ar₁ = Ar₂ = stilbène, et R₈ = alkyle en C₁-C₈, phényle ou un radical hétérocyclique, les deux pouvant être substitués ou non substitués, ou cycloalkyle en C₅-C₁₂.

4. Dicétopyrrolopyrroles fluorescents : 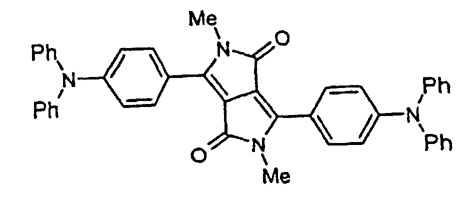 ou 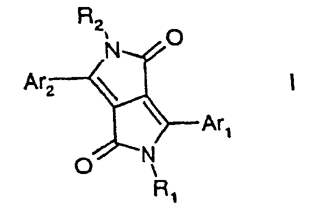 où Ar₁ et Ar₂ sont des groupes 2-naphtyle et
R₁ et R₂ sont des groupes 3,5-diméthylbenzyle,
R₁ et R₂ sont des groupes benzyle,
R₁ et R₂ sont des groupes 2-méthylbenzyle,
R₁ et R₂ sont des groupes 2-phénylbenzyle,
R₁ et R₂ sont des groupes 3-méthylbenzyle,
R₁ et R₂ sont des groupes 4-méthylbenzyle,
R₁ et R₂ sont des groupes 4-phénylbenzyle,
R₁ et R₂ sont des groupes 2-phényléthyle,
R₁ et R₂ sont des groupes 3-phénylbenzyle,
R₁ et R₂ sont des groupes 3-méthoxybenzyle, ou
R₁ et R₂ sont des groupes 3,5-di-tert-butylbenzyle.

5. Dicétopyrrolopyrroles fluorescents selon la revendication 1, 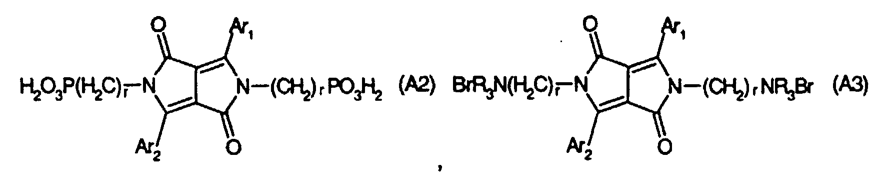 ou 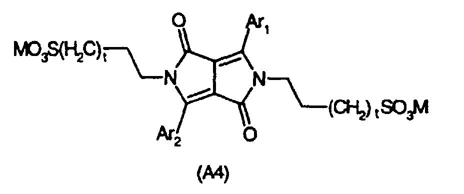 où Ar₁ et Ar₂ sont tels que définis dans la revendication 1, r représente un nombre entier de 2 à 25 ; au lieu de groupes alkyle linéaires, le groupe -(CH₂)ᵣ- peut être remplacé par des groupes alkyle ramifiés ou des groupes aralkyle tels que -(CH₂)ᵣ₁-aryl-(CH₂)ᵣ₂-, r₁ et r₂ étant des nombres entiers compris dans l'intervalle de 0 à 10 ; M représente un ion métallique tel que sodium ou potassium, et t est 1 ou 2.

6. Procédé pour la préparation des composés I selon la revendication 1, par traitement, dans une première étape, du dérivé de DPP de formule V 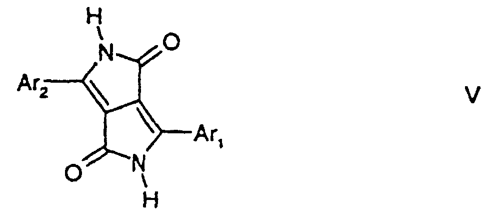 dans laquelle Ar₁ et Ar₂ sont tels que définis dans la revendication 1, avec une base, puis par traitement, dans une seconde étape, du mélange réactionnel obtenu dans la première étape avec un agent alkylant, la base utilisée dans la première étape étant un hydrure, un alcoolate de métal alcalin ou un carbonate, et l'agent alkylant étant un sulfonate, un tosylate, un mésylate, un carbonate, un sulfate ou un composé halogéné de formule (R₁)_{1 ou 2}X, où X représente SO₃-, (*p*-Me-phényl)SO₂-, (2,4,6-triméthylphényl)SO₂-, -CO₃-, -SO₄- ou un halogène, ou un mélange de (R₁)_{1 ou 2}X et (R₂)_{1 ou 2}X, dans laquelle R¹ et R² sont tels que définis da la revendication 1.

7. Procédé pour la préparation des composés selon la revendication 1
(a) par traitement, dans une première étape, du dérivé de DPP de formule VI 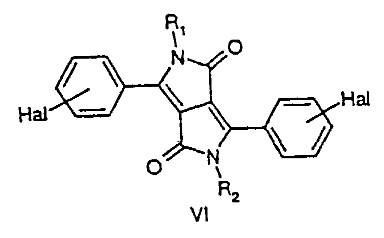 dans laquelle R₁ et R₂ sont de l'hydrogène ou sont tels que définis dans la revendication 1, Hal représente un halogène, avec un agent nucléophile tel qu'une amine secondaire, HNR₈R₉, un thiol, HSR₈ ou HS(O)ₙR₈, un alcool, HOR₁₀, un diséléniure, R₈(O)ₙSe-Se(O)ₙR₈, de préférence en un rapport molaire de DPP VI:agent nucléophile compris dans l'intervalle de 1,2:1 à 0,8:1 ou, si R₂ a la même signification que R₁, dans l'intervalle de 1:2,5 à 1:1, en présence d'un solvant aprotique dipolaire anhydre, et d'une base anhydre en une quantité comprise dans l'intervalle de 0,1 à 15 moles par mole de l'agent nucléophile, à une température comprise dans l'intervalle de 100 à 220°C et sous une pression comprise dans l'intervalle de 100 à 300 kPa, et facultativement en isolant le composé V obtenu,
(b) puis en traitant le composé V (tel que défini dans la revendication 6) obtenu avec une base, puis, dans une seconde étape, en traitant le mélange réactionnel obtenu dans la première étape de (b) avec un agent alkylant,
où, dans la première étape de (b), la base est un hydrure, un alcoolate de métal alcalin ou un carbonate, et l'agent alkylant est un sulfonate, un tosylate, un mésylate, un carbonate, un sulfate ou un composé halogéné de formule (R₁)_{1 ou 2}X, où X représente SO₃-, (*p*-Me-phényl)SO₂-, (2,4,6-triméthylphényl)SO₂-, -CO₃-, -SO₄- ou un halogène, ou un mélange de (R₁)_{1 ou 2}X et (R₂)_{1 ou 2}X.

8. Procédé de coloration de matières organiques de haut poids moléculaire par incorporation des composés DPP I selon l'une quelconque des revendications 1 à 4 par des procédés connus dans la technologie.

9. Composition comprenant
(a) 0,01 à 50 % en poids, par rapport au poids total de la matière organique de haut poids moléculaire colorée, d'un DPP I fluorescent selon l'une quelconque des revendications 1 à 4, et
(b) 99,99 à 50 % en poids, par rapport au poids total de la matière organique de haut poids moléculaire colorée, d'une matière organique de haut poids moléculaire, et
(c) 0 à 50 % en poids d'additifs usuels, par rapport au poids total de (a) et (b).

10. Composition selon la revendication 9, dans laquelle la matière organique de haut poids moléculaire est un polyamide, un polystyrène, de préférence un polystyrène à haute résistance au choc, un polyméthacrylate de méthyle ou un copolymère ABS.

11. Utilisation des DPP I selon l'une quelconque des revendications 1 à 5, pour la préparation d'encres, de colorants, de matières plastiques pigmentées pour revêtements, d'un matériau d'impression sans impact, de filtres colorés, de produits cosmétiques, ou pour la préparation de particules d'encre polymères, de toners, de lasers à colorant et de dispositifs électroluminescents.
